# EUROPEAN PATENT SPECIFICATION

(11) **EP 0 596 901 B1**
(45) Date of publication and mention of the grant of the patent: **09.08.2000**
(21) Application number: 92913946.7
(22) Date of filing: 27.05.1992
(51) Int. Cl.: C12N 15/11, C12N 9/00, A61K 31/70, C07H 21/02

(54) **CHIMERIC tRNA LYS -RIBOZYME MOLECULES**
CHIMERE TRNA LYS-RIBOZYM-MOLEKÜLE
MOLECULES CHIMERIQUES DE RIBOZYMES D'ARNt LYS

(43) Date of publication of application: 18.05.1994
(73) Proprietor: CITY OF HOPE, Duarte California 91010-0269 (US)
(72) Inventor: ROSSI, John J., Glendora, CA 91740 (US); LARSON, Garry P., Covina, CA 91723 (US)
(74) Representative: Hall, Marina
(86) International application number: US9204362
(87) International publication number: WO9324133

(56) References cited:
- EP-A- 0 387 775
- WO-A-90/13641
- WO-A-91/03162
- Second International Conference on Drug Research in Immunologic and Infectious Diseases, November 1989, ROSSI et al., "Ribozymes as therapies for AIDS", Abstract Number 90060615, see entire Abstract.
- International Conference AIDS, National Institute of Allergy and Infectious Diseases, issued June 1989, SARVER et al., "Potential of autocatalytic self-cleaving RNAs as anti-HIV agents", Abstract Number 00286389, see entire Abstract.
- Fifth International Conference on AIDS, June 4-9, 1989, issued June 1989, ROSSI et al., "Synthesis and in vitro function of ribozymes targeted to HIV-1 specific RNA sequences", Abstract Number 90059188, see entire Abstract.
- Journal of Cellular Biochemistry, Supplemental 15D, issued 1991, TAYLOR et al., "Production of anti-HIV-1 ribozymes in E. coli using a novel gene fusion technique", see Abstract CD 217, see entire Abstract.
- Journal of Cellular Biochemistry, Supplement 14A, D 428, issued 1990, ROSSI et al., "Ribozyme mediated intracellular immunity to HIV-1 in CD4 HeLa cells", Abstract Number D 428, see entire Abstract.
- Journal of Cellular Biochemistry, Supplement 15D, issued 1991, SARVER et al., "Strategies for the use of anti-sense ribozymes as anti-HIV-1 therapeutic agents", Abstract Number CD 011, see entire Abstract.
- Biochemistry, Vol. 29, Number 47, issued 27 November 1990, RUFFNER et al., "Sequence requirements of the hammerhead RNA self-cleavage reaction", pages 10695-10701, see entire document.
- Journal of Cellular Biochemistry, Supplemental 13B, issued 1989, ROSSI et al., "Ribozymes targeted to HIV-1 RNA's", Abstract Number G 328, see entire Abstract.
- Proceedings of the National Academy of Sciences of the United States of America, Vol. 88, Number 16, issued 15 August 1991, SIOUD et al., "Prevention of human immunodeficiency virus type 1 integrase expression in E. coli by a ribozyme", pages 7303-7307, see entire document.
- Archives of Biochemistry and Biophysics, Vol. 284, Number 2, issued 01 February 1991, GOODCHILD et al., "Ribozymes that cleave an RNA sequence from human immunodeficiency virus: the effect of flanking sequence on rate", pages 386-391, see entire document.
- Nucleic Acids Research, Vol. 18, Number 1, issued 11 January 1990, HAMPEL et al., "Hairpin catalytic RNA model: evidence for helices and sequence requirement for substrate RNA", pages 299-304, see entire document.
- Science, Vol. 247, issued 09 March 1990, SARVER et al., "Ribozymes as potential anti-HIV-1 therapeutic agents", pages 1222-1225, see entire document.
- EMBO Journal, Vol. 8, Number 10, issued October 1989, BARAT et al., "HIV-1 reverse transcriptase specifically interacts with the anticodon domain of its conjugate primer tRNA", pages 3279-3285, see entire document.
- Science, Vol. 253, issued 19 July 1991, PIEKEN et al., "Kinetic characterization of ribonuclease-resistant 2'-modified hammerhead ribozymes", pages 314-317, see entire document.
- Gene, Vol. 111, Number 2, issued 15 February 1992, WEISS et al., "Synthetic human tRNAlys3 and natural bovine tRNAlys3 interact with HIV-1 reverse transcriptase and serve as specific primers for retroviral cDNA synthesis", pages 183-197, see entire document.

## Description

This invention was made with government support under Grant No. AT 25959 awarded by the National Institutes of Health. The government has certain rights in the invention.

### FIELD OF THE INVENTION

This invention relates to chimeric tRNA^{LYS} ribozyme molecules which compete effectively with tRNA^{LYS} for binding to HIV-1 reverse transcriptase. These chimeric molecules provide a mechanism for delivering inhibitors of HIV-1 transcriptase to the virion particle itself.

### BACKGROUND OF THE INVENTION

It has been demonstrated that the entire tRNA^{LYS} molecule as well as various segments of the tRNA per se are capable specifically of interacting with HIV-1 transcriptase. See Barat, et al. EMBO Journal 8:3279-3285 (1989); Khan, et al. J. Bio. Chem 267:6689-6695 (1992); Weiss, et al., Gene 111:183-197 (1992). Ben-Artzi, Proc. Natl. Acad. Soc. USA 89:927-931 (1992) reports an RNAse cleavage activity associated with HIV-1 reverse transcriptase. This activity is shown to cleave only HIV-1 RNA, not the primer.

Prior to this invention there has been no report of chimeric tRNA^{LYS}-ribozyme molecules.

### SUMMARY OF THE INVENTION

This invention provides novel chimeric tRNA^{LYS}-ribozyme molecules that compete effectively with tRNA^{LYS} for HIV-1 reverse transcriptase binding sites. The chimeric human tRNA^{LYS}-ribozymes inhibit reverse HIV transcription by delivering inhibitors such as ribozymes of HIV-1 reverse transcriptase directly to the virion particle and render it non-functional. The chimeric molecules of this invention thus serve as highly specific non-toxic therapeutic agents.

These chimeric molecules also reveal a novel, site specific RNA cleaving activity of HIV-1.

### EXAMPLES AND DESCRIPTION OF THE FIGURES

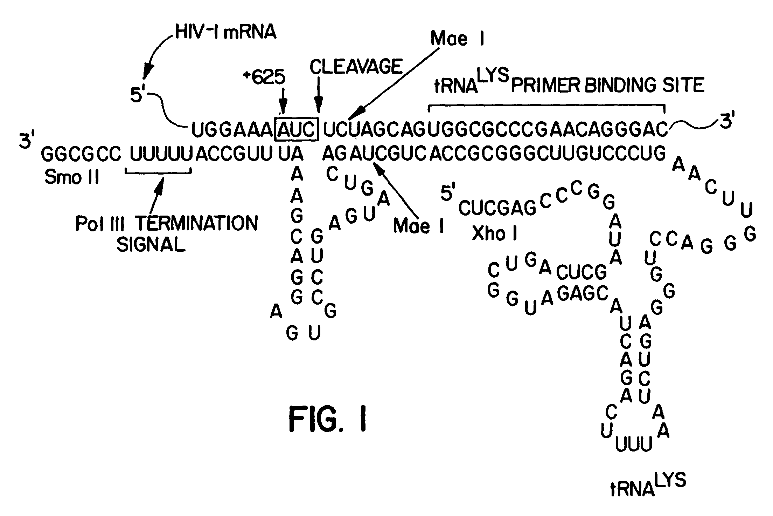

Figure 1 shows the structure of one chimeric ribozyme. This tRNA^{LYS}-ribozyme construct has been cloned into a Blue Script transcription vector using Sacll and Xhol restriction sites. Following linearization at the Sacll site the chimeric RNA can be transcribed in vitro using bacteriphage T-7 RNA polymerase. There is also a Mae I restriction site in between the tRNA and ribozyme moieties, allowing the tRNA to be transcribed independently of the ribozyme.

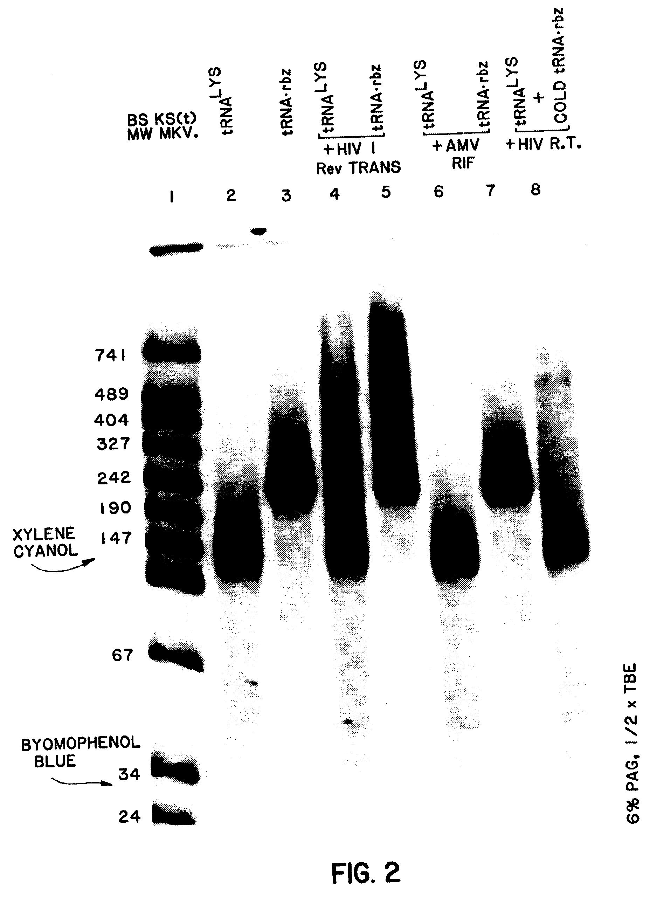

Figure 2. This gel shift experiment shows binding of the chimeric tRNA^{LYS}-ribozyme to HIV-1 reveres transcriptase. The eight lanes of the gel from left to right are:
   1. Molecular weight marker;
   2. tRNA^{LYS} in vitro transcript which has extra bases at both the 5' and 3' ends. The extra 5' bases are from the Blue Script poly linker between the T-7 promoter and the Xhol site. There are six extra nucleotides at the 3' derived from the nucleotides after the CCA of the tRNA to the Mae I site which separates the tRNA from the ribozyme.
   3. tRNA^{LYS}-ribozyme in vitro transcript which has the same extra 5' bases as tRNA^{LYS}, but terminates at Sacll site at the end of the ribozyme moiety.
   4. tRNA^{LYS} transcript incubated with HIV-1 reverse transcriptase.
   5. tRNA^{LYS}-ribozyme transcript incubated with HIV-1 reverse transcriptase.
   6. tRNA^{LYS} transcript incubated with AMV reverse transcriptase.
   7. tRNA^{LYS}-ribozyme incubated with AMV reverse transcriptase.
   8. tRNA^{LYS} with competing, non-radioactively labelled tRNA^{LYS}-ribozyme incubated with HIV-1 reverse transcriptase.

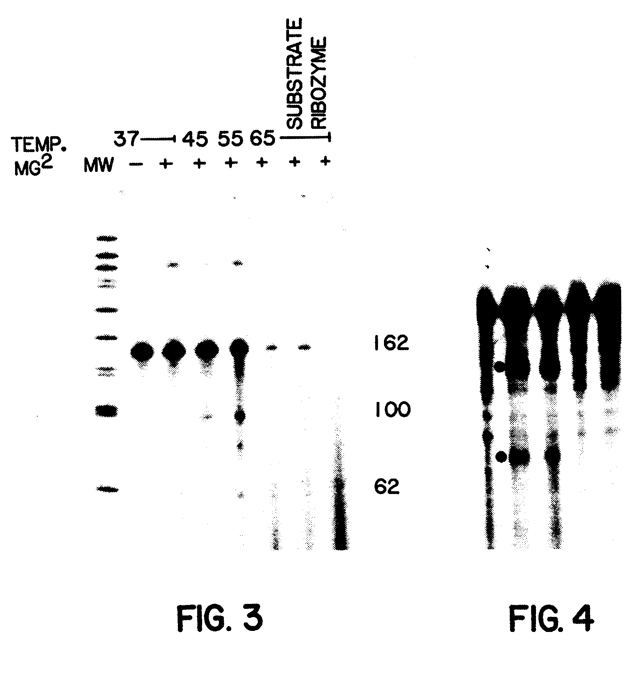

   This Figure 2 shows that the chimeric tRNA^{LYS}-ribozyme specifically binds to HIV-1 reverse transcriptase by a shift in radioactivity when HIV-1 reverse transcriptase is present. Cold tRNA^{LYS}-ribozyme competes with tRNA^{LYS} for binding to HIV-1 reverse transcriptase as indicated by the reduced radioactive shift in lane 8. Figure 3. This experiment demonstrates cleavage
of a 162 nucleotide, radioactively labelled HIV-1 RNA containing the primer binding site plus sequences upstream of this and including the AUC cleavage signal for the ribozyme. The cleavage products are 101 and 61 bases. The extent of cleavage increases with increasing temperature.
Figure 4. Demonstration of the novel RNAse activity of HIV-1 reverse transcriptase when tRNA^{LYS}-ribozyme and HIV-1 primer binding site transcripts are incubated together in the presence of HIV-1 reverse transcriptase. The tRNA^{LYS}-ribozyme is radioactively labelled, and the HIV-1 RNA is non-radioactive. The cleavage products result in the tRNA moiety being separated from the ribozyme moiety. This result also demonstrates that the chimeric tRNA^{LYS}-ribozyme cannot serve as a primer for HIV-1 reverse transcriptase.
   The lanes are, left to right: tRNA^{LYS}-ribozyme alone, tRNA^{LYS}-ribozyme plus HIV-1 reverse transcriptase, no deoxyribonucleoside triphosphates; tRNA^{LYS}-ribozyme plus HIV-1 reverse transcriptase plus deoxyribonucleoside triphosphates; last two lanes same as lane 3 except lane 4 has AMV reverse transcriptase and lane 5 has MLV reverse transcriptase. The black dots mark the HIV-1 reverse transcriptase cleavage products. Unlabelled HIV-1 primer binding site containing 162 nucleotide transcript was present in each lane. None of the reverse transcriptases can utilize the tRNA^{LYS}-ribozyme as a primer since it has 12 nucleotides at the 3' end which cannot base pair with the HIV-1 primer binding site RNA.

### GENERAL DESCRIPTION OF THE INVENTION

Genetic fusions consisting of the entire mature coding sequence or 18 bases of the 3' end of human tRNA^{LYS} have been fused to hammerhead ribozyme containing RNAs with base pairing capabilities to the HIV-1 sequences immediately 5' or upstream of the primer binding site. The 3' terminal 18 nucleotides of the tRNA^{LYS} are complementary to the primer binding site.

These chimeric molecules have been tested in cell free assays for their ability to bind to HIV-1 reverse transcriptase and their inhibitory activity on HIV-1 reverse transcriptase polymerization activity. The ribozyme moiety targets the cleavage of HIV-1 viral RNA at a known hammerhead cleavage site immediately upstream of the primer binding site for initiation of reverse transcription in the HIV-1 viral RNA. The site chosen for initial study, and reported here is an AUC in which cleavage is immediately after the C. This site is absolutely conserved in all HIV-1 isolates sequenced to date. The chimeric RNAs, which are specifically bound by HIV-1 reverse transcriptase should be carried into newly formed HIV-1 virions during viral assembly. The chimeric primers effectively block HIV-1 reverse transcription, making them a novel, highly target specific, and unique anti-HIV-1 therapeutic agent. In addition, the tRNA^{LYS} portion contains within its mature coding sequence the elements required for transcription by human RNA polymerase III, thereby making it feasible to insert the gene, rather than the RNA into human cells.

Studies of the binding of the chimeric molecules to HIV-1 reverse transcriptase, revealed that the complex of chimeric tRNA^{LYS}-ribozyme, or 18 3' nucleotides of tRNA^{LYS}-ribozyme, or tRNA^{LYS} with an extra 6 nucleotides appended to the 3' end, when base paired to the primer binding site signal of HIV-1 RNA, serves as a substrate for a novel ribonuclease activity associated with HIV-1 reverse transcriptase. This activity results in cleavage of the primer at a site very close to the 3' end of the tRNA^{LYS} molecule, CCA-3'. This activity is of unknown function in the viral replication cycle, but may play an important role in the use of chimeric RNAs by freeing the ribozyme moiety from the tRNA moiety such that it can cleave one or both of the viral RNAs encapsidated in the HIV-1 virion.

### GENERAL PURPOSE OR UTILITY OF THE INVENTION

The idea of chimeric tRNA^{LYS}-ribozyme molecules which effectively compete with tRNA^{LYS} for binding to HIV-1 reverse transcriptase is novel. It provides a possible mechanism for specifically delivering inhibitors of HIV-1 reverse transcriptase to the virion particle itself. Such inhibitory agents will render these viral particles non-functional, and thus serve as highly specific, non-toxic therapeutic agents.

It has been demonstrated that the entire tRNA^{LYS} molecule, as well as various segments of the tRNA itself are capable of specifically interacting with HIV-1 reverse transcriptase. No one has shown that chimeric molecules such as the ones described could specifically bind to HIV-1 reverse transcriptase. No other work has described that such molecules are inhibitory to HIV-1 reverse transcriptase polymerase activity. There is one published report of an RNAse cleavage activity associated with HIV-1 reverse transcriptase. This activity was only shown to cleave HIV-1 RNA, not the primer. This activity cleaves twice in the primer binding site, and only substrates paired with tRNA^{LYS}.

The RNA attached to the 3' end of the tRNA^{LYS} need not be a ribozyme, but any extra RNA which can base pair with the HIV-1 target upstream of the primer biding site. If a ribozyme is joined to the tRNA, other cleavage sites such as CUC, or CUA which are on the HIV-1 sequence just to the 3' side (downstream) of the AUC site can be targeted. It is not necessary to make an entire tRNA^{LYS}-ribozyme fusion because it is now known that the last 18 nucleotides of tRNA^{LYS} fused to the ribozyme are also bound by HIV-1 reverse transcriptase. Genetic variants of tRNA^{LYS} which compete better than tRNA^{LYS} for binding to HIV-1 transcriptase are included in the invention.

The ribozyme fusions to tRNA^{LYS} allow specific targeting of the ribozyme to the HIV-1 virion. Since all retroviruses use cellular tRNAs for priming, this invention provides a general strategy for inhibiting other retroviruses as well. Existing ribozyme technology makes use of specific base pairing between ribozyme and target, but this is accomplished by diffusion of the ribozyme until it finds a target RNA. This invention uses well known retroviral packaging pathways to specifically carry the ribozyme into the virion, and get it bound to the correct site on the viral RNA for cleavage.

## Claims

1. A chimeric human tRNA^{LYS}-ribozyme wherein said ribozyme is fused to the 3' end of said human tRNA^{LYS}.

2. A ribozyme according to claim 1 which competes with tRNA^{LYS} for binding to a retroviral reverse transcriptase.

3. A ribozyme according to claim 2 in which the retroviral reverse transcriptase is HIV-1 reverse transcriptase.

4. A construct comprising a ribozyme fused to the coding sequence of the 3' end of human tRNA^{LYS}.

5. A construct as defined by claim 4 in which said ribozyme is a hammerhead ribozyme and said coding sequence comprises at least the eighteen nucleotides of the 3' end of tRNA^{LYS}.

6. A construct according to claim 5 in which said ribozyme moiety has an RNA sequence which base pairs with a retroviral sequence immediately 5' of the primer binding site for the initiation of reverse transcription of retroviral RNA, wherein said ribozyme moiety is selected to target a cleavage site of said RNA sequence immediately 5' of said primer binding site for initiation of the reverse transcription.

7. A construct according to claim 5 in which said ribozyme moiety has an RNA sequence which base pairs with an HIV-1 sequence immediately 5' of the primer binding site for initiation of reverse transcription in HIV-1 viral RNA, wherein said hammerhead ribozyme moiety is selected to target a known cleavage site of said RNA sequence immediately 5' of said primer binding site for initiation of the reverse transcription.

8. A construct as depicted by Figure 1.

9. HIV-1 reverse transcriptase base paired with a chimeric human tRNA^{LYS}-ribozyme wherein said ribozyme is fused to the 3' end of said human tRNA^{LYS}, said chimeric tRNA^{LYS}-ribozyme having an RNA sequence which base pairs with an HIV-1 sequence immediately 5' of the primer binding site for initiation of reverse transcription in HIV-1 viral RNA, said ribozyme moiety being selected to target a known cleavage site of said RNA sequence immediately 5' of the said primer binding site for initiation of reverse transcription in HIV-1 viral RNA.

10. Use of a gene for the manufacture of a medicament for introduction into human cells for the expression of a construct as defined in claim 1 for use in blocking HIV-1 reverse transcriptase.

11. Use of a chimeric ribozyme, said chimeric ribozyme comprising at least the eighteen bases of the 3' end of human tRNA^{LYS} fused to a ribozyme moiety having an RNA sequence which base pairs with an HIV-1 sequence immediately 5' of the primer binding site for initiation of reverse transcription in HIV-1 viral RNA, for the manufacture of a medicament for use as an anti-HIV therapeutic agent to render HIV-1 virion particles non-functional, wherein on being administered to a subject infected with HIV-1 said chimeric ribozyme is specifically delivered to said HIV-1 virion particles so that said chimeric ribozyme base pairs with HIV-1 reverse transcriptase sequence contained in said HIV-1 virion particle.

12. An in vitro method for blocking HIV-1 reverse transcriptase which comprises introduction of a gene which expresses a construct as defined by claim 1 into human cells.

## Patentansprüche

1. Chimäres menschliches tRNA^{Lys}-Ribozym, worin das Ribozym an das 3'-Ende der menschlichen tRNA^{Lys} gebunden ist.

2. Ribozym nach Anspruch 1, welches mit der tRNA^{Lys} um die Bindung an eine retrovirale Transkriptase konkurriert.

3. Ribozym nach Anspruch 2, in welchen die retrovirale reverse Transkriptase die HIV-1 reverse Transkriptase ist.

4. Konstrukt, enthaltend ein an die kodierende Sequenz am 3'-Ende der menschlichen tRNA^{Lys} gebundenes Ribozym.

5. Konstrukt nach Anspruch 4, in welchem das Ribozym ein hammerkopfförmiges Ribozym ist und die kodierende Sequenz mindestens die achtzehn Nukleotide am 3'-Ende der tRNA^{Lys} enthält.

6. Konstrukt nach Anspruch 5, in welchem der Ribozym-Anteil eine RNA-Sequenz hat, die Basenpaarungen mit einer retroviralen Sequenz unmittelbar 5' der Primerbindungsstelle für den Start der reversen Transkription der retroviralen RNA eingeht, worin der Ribozym-Anteil so ausgewählt ist, um eine Schnittstelle der RNA-Sequenz unmittelbar 5' der Primerbindungsstelle für den Start der reversen Transkription gezielt anzusteuern.

7. Konstrukt nach Anspruch 5, in welchem der Ribozym-Anteil eine RNA-Sequenz hat, die Basenpaarungen mit einer HIV-1-Sequenz unmittelbar 5' der Primerbindungsstelle für den Start der reversen Transkription der HIV-1 Virus-RNA eingeht, worin der hammerfkopf-förmige Ribozym-Anteil ausgewählt ist, um eine bekannte Schnittstelle der RNA-Sequenz unmittelbar 5' der Primerbindungstelle für den Start der reversen Transkription gezielt anzusteuern.

8. Konstrukt wie in Figur 1 gezeigt.

9. Mit einem chimären menschlichen tRNA^{Lys}-Ribozym basengepaarte HIV-1 reverse Transkriptase, worin das Ribozym an das 3'-Ende der menschlichen tRNA^{Lys} gebunden ist, das chimäre tRNA^{Lys}-Ribozym eine RNA-Sequenz hat, die mit einer HIV-1 Sequenz unmittelbar 5' der Primerbindungsstelle für den Start der reversen Transkription in der HIV-1 Virus-RNA basenpaart, wobei der Ribozym-Anteil so ausgewählt ist, um eine bekannte Schnittstelle in der RNA-Sequenz unmittelbar 5' der Primerbindungsstelle für den Start der reversen Transkription in der HIV-1 Virus-RNA gezielt anzusteuern.

10. Verwendung eines Gens zur Herstellung eines Arzneimittels zur Einführung in menschliche Zellen zur Expression eines Konstruktes nach Anspruch 1 zur Blockierung der HIV-1 reversen Transkriptase.

11. Verwendung eines chimären Ribozyms, wobei das chimäre, mindestens die achtzehn Basen des 3'-Endes der menschlichen tRNA^{Lys} an den RNA-Anteil gebunden enthaltende Ribozym eine RNA-Sequenz hat, welche mit der HIV-1 Sequenz unmittelbar 5' der Primerbindungstelle für den Start der reversen Transkription in der HIV-1 Virus-RNA basenpaart, zur Herstellung eines Arzneimittels zur Verwendung als therapeutisches Anti-HIV-Mittel, um die HIV-1-Viruspartikel funktionsuntüchtig zu machen, worin, wenn es einer mit HIV-1 infizierten Person verabreicht wird, das chimäre Ribozym spezifisch zu den HIV-1 Viruspartikeln befördert wird, so daß das chimäre Ribozym mit der Sequenz der HIV-1 reversen Transkriptase, die in den HIV-1-Viruspartikeln enthalten ist, basenpaart.

12. *In vitro*-Verfahren zum Blockieren der HIV-1 reversen Transkriptase, welches das Einführen eines, ein Konstrukt gemäß Anspruch 1 exprimierendes Gens in menschliche Zellen umfaßt.

## Revendications

1. ARNt^{LYS}-ribozyme humain chimère, dans lequel ledit ribozyme est fusionné à l'extrémité 3' dudit ARNt^{LYS} humain.

2. Ribozyme selon la revendication 1, qui entre en concurrence avec l'ARNt^{LYS} pour une liaison à une transcriptase inverse de rétrovirus.

3. Ribozyme selon la revendication 2, dans lequel la transcriptase inverse de rétrovirus est la transcriptase inverse du VIH-1.

4. Produit de synthèse comprenant un ribozyme fusionné à la séquence codante de l'extrémité 3' de l'ARNt^{LYS} humain.

5. Produit de synthèse selon la revendication 4, dans lequel ledit ribozyme est un ribozyme à tête de marteau, et ladite séquence codante comprend au moins les dix-huit nucléotides de l'extrémité 3' de l'ARNt^{LYS}.

6. Produit de synthèse selon la revendication 5, dans lequel ledit fragment de ribozyme a une séquence d'ARN qui présente un appariement des bases avec une séquence rétrovirale en position immédiatement 5' par rapport au site de liaison de l'amorce, pour l'initiation de la transcription inverse de l'ARN rétroviral, où ledit fragment de ribozyme est choisi de façon à cibler un site de clivage de ladite séquence d'ARN en position immédiatement 5' par rapport audit site de liaison de l'amorce, pour initiation de la transcription inverse.

7. Produit de synthèse selon la revendication 5, dans lequel ledit fragment de ribozyme a une séquence d'ARN qui présente un appariement des bases avec une séquence de VIH-1 en position immédiatement 5' par rapport au site de liaison de l'amorce pour initiation de la transcription inverse de l'ARN du virus VIH-1, où ledit fragment de ribozyme à tète de marteau est choisi de façon à cibler un site de clivage connu de ladite séquence d'ARN en position immédiatement 5' par rapport audit site de liaison de l'amorce pour initiation de la transcription inverse.

8. Produit de synthèse tel que représenté sur la figure 1.

9. Transcriptase inverse du VIH-1, ayant subi un appariement des bases avec un ARNt^{LYS}-ribozyme humain chimère, où ledit ribozyme est fusionné à l'extrémité 3' dudit ARNt^{LYS} humain, ledit ARNt^{LYS}-ribozyme chimère ayant une séquence d'ARN qui présente un appariement des bases avec une séquence de VIH-1 en position immédiatement 5' par rapport au site de liaison de l'amorce pour initiation de la transcription inverse de l'ARN du virus VIH-1, ledit fragment de ribozyme étant sélectionné de façon à cibler un site de clivage connu de ladite séquence d'ARN en position immédiatement 5' par rapport audit site de liaison de l'amorce pour initiation de la transcription inverse de l'ARN du virus VIH-1.

10. Utilisation d'un gène pour préparer un médicament pour introduction dans des cellules humaines, destiné à l'expression d'un produit de synthèse selon la revendication 1, pour utilisation dans le blocage de la transcriptase inverse du VIH-1.

11. Utilisation d'un ribozyme chimère, ledit ribozyme chimère comprenant au moins les dix-huit bases de l'extrémité 3' d'un ARNt^{LYS} humain fusionné à un fragment de ribozyme ayant une séquence d'ADN qui présente un appariement des bases avec une séquence du VIH-1 en position immédiatement 5' par rapport au site de liaison de l'amorce pour initiation de la transcription inverse de l'ARN du virus VIH-1, pour préparer un médicament destiné à être utilisé en tant qu'agent thérapeutique anti-VIH, dans le but de rendre non fonctionnelles les particules du virion du VIH-1, dans laquelle, après administration à un sujet infecté par le VIH-1, ledit ribozyme chimère est d'une manière spécifique délivré auxdites particules du virion du VIH-1 de façon que ledit ribozyme chimère présente un appariement des bases avec la séquence de la transcriptase inverse du VIH-1 contenue dans ladite particule du virion du VIH-1.

12. Procédé in-vitro de blocage de la transcriptase inverse du VIH-1, qui comprend l'introduction, dans des cellules humaines, d'un gène qui exprime un produit de synthèse selon la revendication 1.
